# EUROPEAN PATENT APPLICATION

(11) **EP 1 281 720 A2**
(43) Date of publication of application: **05.02.2003**
(21) Application number: 02016964.5
(22) Date of filing: 01.08.2002
(51) Int. Cl.: C07K 14/72, C12N 15/12, C12N 15/63, G01N 33/74

(54) **Progestogen receptor from Japanese eel**

(30) Priority: 03.08.2001 JP 2001235725
(71) Applicant: Okazaki National Research Institutes, Okazaki City, Aichi-Pref. (JP)
(72) Inventor: Nagahama, Yoshitaka, Okazaki City, Aichi Prefecture (JP); Ikeuchi, Toshitaka, Okazaki City, Aichi Prefecture (JP); Kobayashi, Toru, Okazaki City, Aichi Prefecture (JP); Todo, Takashi, Sado-gun, Niigata Prefecture (JP)
(74) Representative: Henkel, Feiler, Hänzel

(57) **Abstract**

A polypeptide having an amino acid sequence (a) or (b) given below.
(a) a polypeptide having an amino acid sequence shown by amino acid Nos. 1 to 689 in Sequence Listing No. 2 and (b) a polypeptide which has activity of a progestogen receptor and which is a derivative obtained by way of deletion, substitution, addition or insertion of a part of the amino acid sequence in the above (a). Corresponding DNA gene and transgenic cell as well as a method for detecting a disruptor using this transgenic cell are also disclosed.

## Description

### Background of the Invention

### (1) Field of the Invention

The present invention relates to transgenic cells and a method for detecting disruptors by using said transgenic cells.
More specifically, the invention relates to transgenic cells utilizing foreign reporter genes, and a method for detecting disruptors by using said transgenic cells.

### (2) Related Art Statement

The hormones are substances which are produced and secreted from specific cells (mainly endocrine cells, and a part of nerve cells) in response to information from the interior and the exterior of the living bodies and which transmit such information to other cells via body fluids. The hormones are broadly classified into three main kinds : peptide hormones such as insulin and glucagon, steroid hormones such as androgen and estrogen, and amine hormones such as adrenalin. Every hormone binds to a hormone receptor inside a target cell, changes its physical property and induces changes in reaction inside the cell. The amine hormones and the peptide hormones act mainly on cell membranes, and control the metabolism inside cells through functional changes of the cell membranes or adjustment of the production of second messengers, whereas the steroid hormones mainly control the expression of the genetic information through binding to receptors inside the cytoplasm or nuclei.

Such hormone information constitutes important factors to ensure normal life of the living things, such as functional expression, homeostasis, etc. Particularly, the progestogen plays an important role in maintaining the pregnancy, and is an important factor involved in the final muturation in the case of oviparous living things.

Chemical substances that structurally resemble such hormones sometimes disrupt actions of the intrinsic hormones or make them to abnormally act, thereby influencing human bodies and/or generative functions. The artificial organic compounds structurally resembling the hormones are called environmental hormones.

Different from the normal hormones which are decomposed in livers and discharged outside soon after they terminate their functions, environmental hormones are unfavorably difficult to be decomposed, and are dissolve in oil and hardly discharged from the human bodies.

In addition to the above, the environmental hormones have serious problems in that their extremely small amounts function like the normal hormones, cause abnormality against the generative functions regarding the species survival, extreme influences specially upon specific time periods such as fetal stage and abnormality regarding to the foundation of the life such as abnormality in the immune system or abnormality in the behavior.

Under the circumstances, it is an urgent task to search and grasp how much the atmospheres, waters, soils, etc. are contaminated with the environmental hormones.

In general, an equipment called "gas chromatograph mass spectrometer" is known for the analysis of minute amounts of analytes. According to this analysis system, an extraction treatment is effected as a pretreatment that a sample such as soil or water is chemically treated to remove as much ingredients other than a target one as possible. Thereafter, the sample is passed through the analyzer together with a gas, which is subjected to finer separation and measurement of the target ingredient.

However, the above gas chromatograph mass spectrometer requires the extraction operation to preliminarily remove as much the ingredients other than the target one as possible.

If the ingredient to be measured is in a minute amount, the analysis precision is largely deteriorated unless the extraction operation is skillfully performed. Although the quantitative measurement and detection by the gas chromatograph are excellent in examining known substances of which disrupting actions are known. With respect to unknown substances, it is impossible to detect whether such unknown substances exhibit disrupting functions or not. Further, it is impossible to examine how strong the unknown substances act as compared with internal hormones.

Besides, there are many substances that disturb the endocrine system despite of their structural dissimilarity. Such substances can be detected only by checking their actual influences upon living bodies or examining their binding ability to corresponding receptors. Further, problems are that it is troublesome and takes a long time to confirm the influences upon the living bodies, and only examination of the binding ability cannot clarify whether the substances actually function in cells in an accelerated manner or inhibited manner.

Therefore, a detection method that can detect disturbing substances simply even in minute amounts has been desired. However, a method for detecting such disruptors has not been known up to now.

### Summary of the Invention

It is therefore an object of the present invention to provide a disrupting substance-detecting method which can accurately detect a minute amount of progestogen function-disrupting substance.
It is another object of the present invention to provide polypeptides having activity as progestogen receptors.
It is a further object of the present invention to provide DNA genes encoding proteins exhibiting having activity as such progestogen receptors.
It is a still further object of the present invention a transgenic cell in which a progestogen receptor expression vector and a foreign reporter gene having a gene promoter, which is a target of the progestogen, are introduced in a culture cell.

In order to accomplish the above object, the present inventors bound a group of genes acting upon progestogen to foreign reporter genes, and made strenuous investigations of transgenic cells containing said gene group. As a result, the inventors discovered the present invention. More specifically, the inventors isolated two kinds of cDNA though to encode progestogen receptors, prepared polypeptides according to them, and examined binding ability of the polypeptides to the progestogen.

The polypeptide according to the present invention is a polypeptide having an amino acid sequence (a) or (b).
(a) a polypeptide having an amino acid sequence shown by amino acid Nos. 1 to 689 in Sequence Listing No. 2
(b) a polypeptide which has activity of a progestogen receptor and which comprises a derivative, by way of amino acid deletion, substitution, addition or insertion of the amino acid sequence in the above (a)

The DNA gene according to the present invention is a DNA sequence (a) or (b) given below.
(1) a DNA sequence having nucleotide sequence Nos. 1-2582 in Sequence Listing No. 1
(b) a DNA sequence which hybridizes under stringent condition to the DNA sequence in (a) and which encodes the protein having activity of the progestogen receptor.

The transgenic cell according to the present invention comprises a culture cell, and a progestogen receptor-expression vector containing the above DNA sequence and a foreign reporter gene introduced in the culture cell, the foreign reporter gene having a promoter for the gene, which is a target of said progestogen and the progestogen receptor, inserted.

According to a preferred embodiment of the transgenic cell of the present invention, the foreign reporter gene is at least one gene selected from a luciferase gene, a chloramphenicol acetyl transferase (CAT) gene and a β-galactosidase gene.

According to another preferred embodiment of the transgenic cell of the present invention, the progestogen receptor-expression vector of the present invention is a progestogen receptor α-expression vector or a progestogen receptor β-expression vector.

The present invention also relates to a method of detecting a disruptor. The disruptor-detecting method comprises the steps of contacting the above transgenic cell with a material to be examined, detecting the expression of the foreign reporter gene and thereby detecting a disruptor in the material.

According to a preferred embodiment of the disruptor-detecting method of the present invention, the intensity of the expression of the foreign reporter gene is measured based on the intensity of light emission of luciferase.
These and other objects, features and advantages of the invention will be appreciated when taken in conjunction with the attached drawings, with the understanding that some modifications, variations and changes could be made by the skilled in the art to which the invention pertains.

### Brief Description of the Invention

For a better understanding of the invention, reference is made to the attached drawing, wherein:

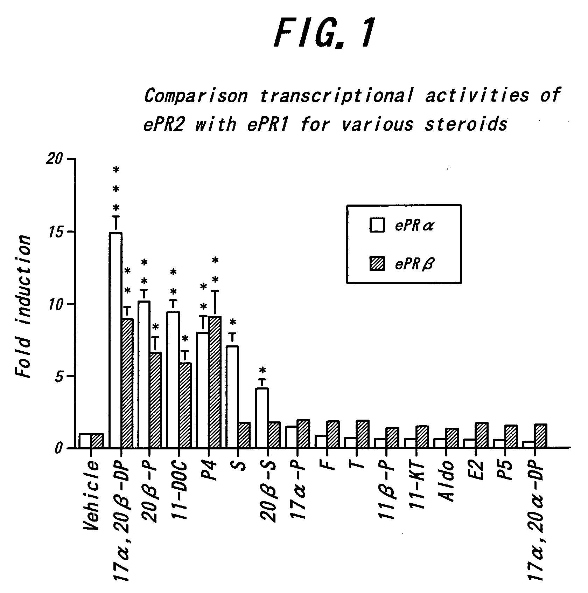

Fig. 1 is a graph showing detected results of various steroids in one example of the present invention.

### Detailed Description of the Invention

The DNA gene according to the present invention is a DNA sequence (a) or (b) : (1) a DNA sequence having nucleotide sequence Nos. 1-2582 in Sequence Listing No. 1 and (b) a DNA sequence which hybridizes under stringent condition to the DNA sequence in (a) and which encodes the protein having activity of the progestogen receptor. The DNA gene expresses the progestogen receptor. Further, the DNA gene according to the present invention can be also used as a probe for searching an analogous protein having the activity of the progestogen receptor.

In order to obtain the DNA gene according to the present invention, RNAs are extracted from a testis, and mRNA is purified from said RNAs by using an ordinary method such as an affinity chromatography.

The thus obtained mRNA is inversely transcripted to cDNA with a reverse transcriptase. In order to screen from the cDNA the DNA gene which can express the intended progestogen receptor protein, an amplification method such as PCR method is preferably used from the standpoint that such enables the searching in a short time.

A primer used in the PCR method can be designed and synthesized in light of a sequence common among known steroid receptors. Target cDNA sequences can be amplified with the above primer by using as a template the cDNA obtained with the reverse transcriptase.

The target cDNA sequence can be determined by utilizing an ordinary method such as a terminator method.

According to the gene recombination technique, a specific site of the fundamental DNA sequence can be artificially mutated without changing fundamental characteristics of the DNA sequence. The DNA gene having the natural nucleotide sequence provided by the present invention can be modified to DNA sequences having equivalent or improved characteristics as compared with those of the natural gene by way of artificial deletion, substitution, addition or insertion of nucleotide(s). The present invention includes such variant DNA genes.

The DNA genes falling in the present invention which are derivatives obtained by way of deletion, substitution, addition or insertion with respect to the nucleotide sequence in the Sequence Listing No. 1 are intended to mean genes in which not more than 10, preferably not more than 7 and more preferably not more than 3 nucleotides are deleted, substituted or added in the nucleotide sequence shown in the Sequence Listing No. 1. Such DNA genes exhibit not less than 90%, preferably not less than 95%, more preferably not less than 98% homology to the gene shown in Sequence Listing No. 1. Such DNA genes form hybrids with that shown in Sequence Listing No. 1 under stringent conditions. Such genes fall in the genes according to the present invention, so long as the former bind to the progestogen and have the activity of the progestogen receptors.

The transgenic cell according to the present invention is obtained by introducing into a culture cell a progestogen receptor expression vector and a foreign reporter gene into which is inserted a promoter for a gene that is a target of the progestogen and the progestogen receptor.

First, how to construct the progestogen receptor expression vector will be explained. For example, the expression vector may be constructed by amplifying cDNA fragments adapted for translating a progestogen receptor α or a progestogen receptor β from a cDNA pool being prepared from a tissue of a testis or the like by PCR using primers which introduced EcoRI site, and ligating PCR resultant with an expression vector, pcDNA 3.1(+) manufactured by Invitrogen Co., Ltd. at the EcoRI site by using a DNA ligase.

Results similar to those in the present invention can be realized by placing the progestogen receptor in a nucleus. However, it is extremely difficult to obtain the similar results by placing the receptor in the nucleus as compared with the case where the progestogen receptor is synthesized by the expression vector within the cell. From this point of view, the progestogen receptor expression vector is constructed and introduced into the cell.

The progestogen receptor expression vector is introduced to express the progestogen receptor in the nucleus of the cell. The progestogen receptor binds to the disruptor to form a progestogen receptor-disruptor complex. Therefore, the disruptor acts in the same manner as the progestogen or exerts an inhibiting function against the progestogen. By utilizing such a function and mechanism, the transgenic cell according to the present invention can be used to detect the disruptor.

According to another aspect of the present invention, a plurality of progestogen receptor expression vectors such as the progestogen receptor α expression vectors or the progestogen receptor β expression vectors may be used as the above progestogen receptor expression vector. By using the plural progestogen receptor expression vectors, the disruptors can be captured at a higher accuracy. That is, even the disruptor that fails to bind to the receptor α may bind to other receptor(s) the progestogen receptor β, so that the disruptors may be detected without failure.

As the progestogen receptor expression vector, one originated from a verbrate may be used. For example, a progestogen receptor originating from a human being, a mouth, an eel or the like may be used. From the standpoint of view that two kinds of progestogen receptor expression vectors can be obtained, the progestogen receptors originating from the eel may be preferably recited.

Next, how to construct the foreign reporter gene to which the promoter for the gene that is to be targeted by the progestogen and the progestogen receptor is inserted will be explained.

First, a promoter region for a gene which is to be targeted by an progestogen and the progestogen receptor (hereinafter referred to as "target gene") is obtained. Since there are plural target genes for the progestogen receptor, any of the target sequences may be used. As the promoter region for the target gene, a vector such as pMSG manufactured by Pharmacia Co., Ltd. may be utilized. Since this vector contains a promoter called LTR of a virus MMTV, this region only may be cut out with two kinds of restriction enzymes : Nhe I and Hind III.
Particularly, MMTV-LTR may be preferably used, because it has a wide applicability.

In the present invention, needless to say, various promoters can be used. However, a promoter of a certain target promoter is expressed in muscle only, and a transcription factor specific to the muscle needs to be coexistent. Therefore, if such a promoter is used, various transcription factors or a culture cell system originating from the muscle is required in this case.

As mentioned above, the promoter region can be obtained. The promoter region of the target gene is bound to the foreign reporter gene according to an ordinary method. For example, cohesive ends are formed for the promoter region and the foreign reporter gene by using a restriction enzyme, and then bound to each other with a DNA ligase or the like. For example, it may be that a promoter called "MMTV-LTR" is cut out from a pMSG vector in Pharmacia Co., Ltd., and their ends are made blunt with Klenow fragment of DNA polymerase after using two restriction enzymes : Nhe I and Hind III, and can be bound, with use of a DNA ligase, to Sma I-digested ends of a luciferase expression vector commercially available from Promega Co., Ltd. which produce blunt ends of a protein translation portion of a luciferase (corresponding to the foreign reporter gene).

The progestogen target gene-bonded foreign reporter gene can be constructed in this manner.

The foreign reporter gene is not particularly limited, but such a foreign reporter gene of which mRNA or its expressed protein is easily confirmed is preferred.

From the standpoint that speedy confirmation can be made at high sensitivity, luciferase, chloramphenicol acetyl transpherase (CAT) and β-galactosidase may be recited as the foreign reporter gene.

The transgenic cell according to the present invention is obtained by introducing the progestogen receptor expression vector and the foreign reporter gene into which the progestogen target gene promoter is inserted into the culture cell. In order to enable the foreign reporter gene to effectively reach the translation, another vector may be introduced into the transgenic cell. In the present system, it is possible that additional genes, which are other reporters or transcription factors and so on, can be contransfected trangently. In this case, internal control vector such as pRL-TK, which contains the seapansy (*Renilla reniformis*) luciferase gene with the herpes simplex virus thymidine kinase promoter in order to normalize for variation in transfection efficiency, may be used.

An ordinary method can be used to introduce the foreign reporter gene and the vector into the culture cell. As the gene-introducing method, a phage transduction method, a plasmid gene introduction method, a microinjection method, an electroporation method and a calcium phosphate method may be recited, for example.

Whether the foreign reporter gene and so on are introduced into the culture cell or not can be confirmed, while two kinds of samples : progestogen and a solvent into which the progestogen is dissolved are prepared. That is, if they are not introduced into the cell, light emission with luciferase is detected in the progestogen-added group at an equivalent level to that in the solvent-added group. Thus, the introduction of the foreign reporter gene and the vector into the cell can be confirmed.

The foreign reporter gene and the vector are introduced into the culture cell. If the vector is taken into the cell, it moves into the nucleus. Although it does not selectively move into the nucleus, it can certainly move into the nucleus.

Next, the method for detecting the disruptor according to the present invention will be explained.
According to the disruptor-detecting method of the present invention, the transgenic cell of the invention is contacted with a material to be examined, and the expression of the foreign reporter gene is examine to detect the disruptor.

The principle of the disruptor-detecting method according to the present invention will be first explained. This principle is based on the utilization of the above-mentioned transgenic cell. When the disruptor contacts the transgenic cell, it passes a cell membrane, binds to the progestogen receptor inside the nucleus and thereby forms a progestogen receptor-disruptor composite. It is considered that this composite causes a change in the conformation of the gene, and this composite acts upon a factor necessary for the expression and for the expression of the foreign reporter gene to which the promoter is inserted. Thereby, the foreign reporter gene downstream the promoter is expressed.

Therefore, when the expression of the foreign reporter gene is checked, whether the material to be examined exhibits a progestogen function or a progestogen-interrupting function or not can be judged.

Regarding the transgenic cell, see the above explanation. As mentioned above, the foreign reporter gene is not particularly limited.

From the standpoint that definite and speedy confirmation of the disruptor can be made at high sensitivity, luciferase gene, chloramphenicol acetyl transpherase (CAT) gene and β-galaxxidase gene may be recited as the foreign reporter gene.

Materials to be examined which are presumed to contain disruptors are not particularly limited. For example, samples taken from the atmosphere, waters such as rivers and sea water, foods, body fluids of animals and plants, soils, etc. may be used as the materials to be examined. In this case, since it is likely that if such water is used as it is, the culture cells are destroyed with bacteria existing therein, the culture cells can used as being coarsely extracted with an organic solvent such as ethanol, for example.

When the luciferase gene is used as the foreign reporter gene, whether the foreign reporter gene is expressed or not can be quantitatively detected based on the intensity of the light emission of the luciferase protein.

When the chloramphenicol acetyl transpherase (CAT) gene is used as the foreign reporter gene, whether the foreign reporter gene is expressed or not can be quantitatively detected by an enzyme immunoassay or based on the amount of radiations of radioactively labeled chloramphenicol into which an acetyl group is introduced.

When the β-galactosidase gene is used as the foreign reporter gene, whether the foreign reporter gene is expressed or not can be quantitatively detected by the enzyme immunoassay or based on the intensity of a color reaction using a chromophoric substrate.

### (Examples)

In the following, the present invention will be explained in more detail, but the invention should not be interpreted as limited to these examples.

cDNAs of eel progestogen receptors were obtained by isolating mRNA from an eel testis, constructing cDNA, and amplified using the cDNA as template with α-specific primers and β-specific primers according to the PCR method, respectively.

More specifically, RNA was extracted from the eel testis by using ISOGEN (manufactured by WAKO Pure Pharmaceutical Co., Ltd), and mRNA was purified with Oligotex-dT30 (manufactured Takara Co., Ltd.). cDNA was obtained from the thus obtained mRNA with a reverse transcriptarase (Gibuko), and several kinds) of cDNA fragments were obtained from the cDNA as a template according to the PCR method by using primers designed and synthesized in light of amid acid sequences common among known steroids. The thus obtained cDNA fragments were inserted into cloning vector pGEM-T-easy (Promega Co., Ltd.), and the sequence of the cDNA was determined by a DNA sequencer. Since two kinds of clones having high homology to the known progestogen receptor was discovered, respectively specific primers for them were designed and synthesized, and an entire cDNA was obtained by using a Marathon cDNA amplification kit (Clonetec Co., Ltd.) according to a 5'- and 3'-RACE methods. Its sequence was determined in the same way.

The sequence of the thus obtained gene is shown in Sequence Listing No. 1. The amino acid sequence of the polypeptide expressed by this gene is shown in Sequence Listing No. 2. This novel peptide is called progestogen receptor β, and its receptor activity will be explained in Example 2.

As mentioned later, this receptor β is a very important protein in detecting a disruptor at a high sensitivity. That is, by using the plural progestogen receptor expression vectors, the disruptors can be captured at a higher accuracy. That is, even the disruptor that fails to bind to the receptor α may bind to other receptor(s) the progestogen receptor β, so that the disruptors may be detected without failure.

### Example 2

As progestogen receptor expression vectors, an eel progestogen receptor α expression vector and an eel progestogen receptor β expression vector were used. The eel progestogen receptors were obtained by isolating mRNAs from an eel testis, constructing cDNA, and amplifying them with α-specific primers and β-specific primers according to the PCR method, respectively.

The luciferase gene was used as a foreign reporter gene. A commercially available vector (Promega Co., Ltd.) was used as a luciferase expression vector. The foreign reporter gene to which a promoter was inserted was prepared by incorporating a steroid-responsive promoter originated from a long terminal repeat of a murine mammary tumor virus of the upstream of luciferase gene.

Into a 293 cell (culture cell) were transfected 50 ng of the progestogen reporter expression vector and 250 ng the luciferase reporter vector inside each hole of a commercially available 24-well culture plate. To the culture cell was added a steroid at 100 nM concentration, which was cultured for 48 hours. More specifically, substances (progestogen-like substances) having the progestogenic function or an inhibiting ability were detected with use of (1) 17α, 20β-dihydroxy-4-pregnen-3-one (17α, 20β-DP), (2) 20β-hydroxyprogesterone (20β-P), (3) 11-deoxycorticosterone (11DOC), (4) progesterone (P4), (5) 17α, 21-dihydroxyprogesterone (S), (6) 17α, 20β, 21-trihydroxyprogesterone (20β-S), (7) 17α-hydroxyprogesterone (17α-P), (8) cortisol (F), (9) testosterone (T), (10) 11β-hydroxyprogesterone (11β-P), (11) 11-ketotestosterone (11-KT), (12) aldosterone (Aldo), (13) estradiol-17β (E2), (14) pregnenolone (P5), (15) 17α, 20α-dihydroxy-4-pregnen-3-one (17α, 20α-DP).

Results obtained are shown in Fig. 1. In Fig. 1, abbreviations such as 17α, 20α-DP denote the various steroids as mentioned above. Further, ePRα and ePRβ denote the groups in which the eel progestogen receptor α expression vector and the eel progestogen receptor β expression vector are introduced, respectively.

On the other hand, *, ** and *** in Fig. 1 show significant difference from vehicle groups (hormone solvent, ethanol added) at P<0.05, P<0.01 and P<0.001, respectively. The intensity of light emission of each of the vehicle groups (hormone solvent, ethanol added) is shown. More specifically, intensity of light emission of each hormone vehicle is shown by the number of times with respect to the intensity of the light emission of the vehicle only.

It is seen from these results that a minite amount of each of the various hormones can be detected at a high sensitivity.

The transgenic cell according to the present invention has an advantageous effect that it can sensitively react upon contacting a minute amount of the disruptor.

The disruptor-detecting method according to the present invention has an advantageous effect that a minute amount of the disruptor can be accurately detected.

Further, according to the disruptor-detecting method of the present invention, whether the substance has a disrupting function or not can be detected, irrespective of being known or unknown.

According to the disruptor-detecting method of the present invention, not only whether the substance has the disrupting function or not but also how strong the power of the disruptor is as compared with that of the inherent endohormone can be examined.

<110> Chairman of Okazaki National Joint Research Organization
<120> Progestogen receptor polypeptide, transgenic cell into which a gene encoding
the polypeptide is introduced, and a method for detecting a disruptor by using this
transgenic cell
<130> U2001P022
<160> 2
<210> 1
<211. 2582
<212> nucleic acids
<213> testis of Japanese eel
<400> 1
gcggcgcgtc tcgtttcacc ctaatttcta caggtgaagg actccgactt cttgtggtat 60
tgccgatgaa ctctccaaat tagtatcgtc tacatcaacg gaaaaaatac ttccgggacc 120
tattcttttt cttttttttt atttataggt agccatatgt caatgggata gttatgttac 180
attgcgcgtt aatcttctac aataccagtg ctttaacttg gctttaatca cagccgaatc 240
atttgtataa ttacactaat tgtgttttaa atacatggaa actaatagtt tacaggttga 300
tgcatataaa aagtaccgta aatggtttat tcaataaata acaaattgaa aagcaagtcg 360
taacaacgaa agaggacaac ttggaaaata gtttgttcta acgtggcaga aaacgtccag 420
ttgctgagcg ggttgagatg atgctcactg tttgacgaat gccagtggca gaactgcatt 480
ttctgtgctc aaaacggttc cagcatggac agcgtcagaa aagacaagtc aggagcaacg 540
tctccaacag cgtcacgccc tagggacaca tttatgaaaa ctgacaacga tttgaccgaa 600
gggttttcag attctacttc taactacatg gcaggatctt gcagtacagc taattcaatg 660
tactcgctta gcggcgtctc ttccacaatg cgcaattctg gaaatgcgct tagcggcgtc 720
tcttccacaa tgcgcaattc tggaaatgtg gacacgacgc gccacggagc caactcaact 780
aatgacacga cagagagcgt tgcagttgct gaaaacgctg ctagatataa tgacagtcga 840
gaagctggaa gaacagagtc aaaggccaac aattctcctt ggacgacatc attggcagat 900
aacgaaggac tggcgctgcc tccagcctca gggtcaaaag caagtttgag cggagtatcc 960
tcttcgtcag ttggaaactg caaatttatt aaagatgagc aggactcgtc cagttcaatg 1020
gaaccccagt ctccttactt tcatccatca ggaaatataa cgacttcgaa cagctcgtat 1080
ggaacatgtg aggaggattc agcgacgcat caccccccac atatgtttac ggactataat 1140
cggacgactg cattgcctct cattcccgaa atcacggaag atcaatttag ttttccctat 1200
cctgtcggtg aagttgtcgc gaatagctgc ctgacgggat acggacagcg gtcgccgcag 1260
aattcactta ggtttaagag cgagctttgc aagctatcgc ttccgacctc gtctccagaa 1320

tctcaaagtt ggtgtcagtc aacaggtctg tcggaggatc agcacttcga gactggctat 1380
ctgcctcccg gggagatccg caatatatgt gagacacaca attccttgaa gtcgcacagc 1440
gtgtatatgg ggatgctgtc ccagaaattt tgcctcatct gtggggatga agcgtctgga 1500
tgtcactacg gggttctcac gtgcggcagc tgcaaagtat tctacaagag agccgttgaa 1560
gggcaccaga actacctttg tgccgggaga aacgactgca tcgtggacaa gatccgcagg 1620
aagaactgcc cggcgtgtcg gctgaggaag tgctaccagg ccggaatgac cctgggaggt 1680
cggaagatga agaagctcag tgctttgaag gttctgggtt tgacccagtc cctggccgtc 1740
cgctcgccgc tgggtgcgtc ctacgagggc caggccctgg ccacccttcc cagcatgcct 1800
atggtcagag agctgcagtt cacccctcag atcctcagca tcctggagaa catcgagccg 1860
gagaccgtgt actcggggta cgacgccacc cagcccgaaa cgccccacct cctgctcaac 1920
agcctgaacg ggctctgcga gaggcagctg ctctggatcg tccgctggtc aaagtccctt 1980
ccaggatttc gcagtttaca tatcaatgac caaatgacgc tcatccagta ctcctggatg 2040
agtctgatgg tgttctcact gggatggagg tcatttcaga atgtgaccag agaatttctg 2100
tacttcgcac cagatctcat tctcggcgaa gagaaaatga ggaattctcc catctctgac 2160
ttgtgcatgg caatgcaaat cattccacaa gcatttgata accttcaggt gaccaaggag 2220
gagtttttgt gcatgaaggt cttgctgtta ctcaacactg tgccgctgga aggcctgaga 2280
agccaggccc agttcgacga gatgcggcac ggctacatcc gcgagctcac caaggccatc 2340
cagctgacgg agaggggtgt gatggccagc tcccagcggt tctaccacct gaccaagctc 2400
atggacgcca tgcacgagat cgtgaggaag gtaaacctgt actgtctgag caccttcatc 2460
caggccgaag ccatgcaggt ggagttccca gagatgatgt cagaggtcat aacctcccag 2520
ctgcccaagg ttctggcagg catggtgagg cccctcctct ttcacaaaaa atgatgccct 2580
gc 2582

<210> 2
<211> 689
<212> amino acids
<213> Deduced from Sequence Listing
<400> 2
mdsvrkdksg atsptasrpr dtfmktdndl tegfsdstsn ymagscstan smyslsgvss 60
tmrnsgnals gvsstmrnsg nvdttrhgan stndttesva vaenaarynd sreagrtesk 120
annspwttsl adneglalpp asgskaslsg vssssvgnck fikdeqdsss smepqspyfh 180
psgnittsns sygtceedsa thhpphmftd ynrttalpli peitedqfsf pypvgevvan 240
scltgygqrs pqnslrfkse lcklslptss pesqswcqst glsedqhfet gylppgeirn 300

icethnslks hsvymgmlsq kfclicgdea sgchygvltc gsckvfykra veghqnylca 360
grndcivdki rrkncpacrl rkcyqagmtl ggrkmkklsa lkvlgltqsl avrsplgasy 420
egqalatlps mpmvrelqft pqilsileni epetvysgyd atqpetphll lnslnglcer 480
qllwivrwsk slpgfrslhi ndqmtliqys wmslmvfslg wrsfqnvtre flyfapdlil 540
geekmrnspi sdlcmamqii pqafdnlqvt keeflcmkvl lllntvpleg lrsqaqfdem 600
rhgyireltk aiqltergvm assqrfyhlt klmdamheiv rkvnlyclst fiqaeamqve 660
fpemmsevit sqlpkvlagm vrpllfhkk 689

## Claims

1. A polypeptide having an amino acid sequence (a) or (b) given below.
(a) a polypeptide having an amino acid sequence shown by amino acid Nos. 1 to 689 in Sequence Listing No. 2
(b) a polypeptide which has activity of a progestogen receptor and which is a derivative obtained by way of deletion, substitution, addition or insertion of a part of the amino acid sequence in the above (a)

2. A DNA gene represented by (a) or (b) given below.
(1) a DNA sequence having nucleotide sequence Nos. 1-2582 in Sequence Listing No. 1
(b) a DNA sequence which hybridizes under stringent condition to the DNA sequence in (a) and which encodes a protein having activity of a progestogen receptor.

3. A transgenic cell comprises a culture cell, and a progestogen receptor-expression vector containing the DNA sequence set forth in claim 2 and a foreign reporter gene introduced in the culture cell, the foreign reporter gene having a promoter for the gene, which is a target of said progestogen and the progestogen receptor, inserted.

4. The transgenic cell set forth in claim 3, wherein the foreign reporter gene is at least one gene selected from a luciferase gene, a chloramphenicol acetyl transferase (CAT) gene and a β-galactosidase gene.

5. The transgenic cell set forth in claim 3 or 4, wherein the progestogen receptor-expression vector is a progestogen receptor α-expression vector or a progestogen receptor β-expression vector.

6. A method of detecting a disruptor, comprises the steps of contacting the transgenic cell set forth in any one of claims 3-5 with a material to be examined, detecting the expression of the foreign reporter gene and thereby detecting a disruptor in the material.

7. The disruptor-detecting method set forth in claim 6, wherein existence of the expression of the foreign reporter gene is measured based on the intensity of light emission of luciferase.
